# EUROPEAN PATENT SPECIFICATION

(11) **EP 3 409 184 B1**
(45) Date of publication and mention of the grant of the patent: **06.12.2023**
(21) Application number: 18171530.1
(22) Date of filing: 09.05.2018
(51) Int. Cl.: A61B 1/00

(54) **ENDOSCOPE CONTROL DEVICE, ENDOSCOPE MAINTENANCE SUPPORT METHOD, AND ENDOSCOPE MAINTENANCE SUPPORT PROGRAM**
ENDOSKOPSTEUERUNGSVORRICHTUNG, ENDOSKOPWARTUNGSUNTERSTÜTZUNGSVERFAHREN UND ENDOSKOPWARTUNGSUNTERSTÜTZUNGSPROGRAMM
DISPOSITIF DE COMMANDE D'ENDOSCOPE, PROCÉDÉ DE SUPPORT DE MAINTENANCE ENDOSCOPE ET PROGRAMME DE SUPPORT DE MAINTENANCE D'ENDOSCOPE

(30) Priority: 31.05.2017 JP 2017108461
(43) Date of publication of application: 05.12.2018
(73) Proprietor: FUJIFILM Corporation, Tokyo 106-8620 (JP)
(72) Inventor: TAKAHIRA, Masayuki, Kanagawa, 258-8538 (JP); KAWAMURA, Noriko, Kanagawa, 258-8538 (JP); SUZUKI, Issei, Kanagawa, 258-8538 (JP)
(74) Representative: Klunker IP Patentanwälte PartG mbB

(56) References cited:
- EP-A1- 2 606 814
- EP-A1- 2 698 096
- US-A1- 2005 103 354
- US-A1- 2012 130 160

## Description

### BACKGROUND OF THE INVENTION

### 1. Field of the Invention

The present invention relates to an endoscope control device, an endoscope maintenance support method, and a non-transitory computer readable recording medium storing an endoscope maintenance support program.

### 2. Description of the Related Art

Medical apparatuses including an endoscope apparatus are required not only to improve the performance of the apparatuses themselves but also to improve after-sales service to perform maintenance, such as periodic inspection or repair.

JP2005-122707A discloses that the operation state information of a medical apparatus including an endoscope apparatus is collected and maintenance of the medical apparatus is performed using the operation state information.

JP2006-043152A discloses a system that collects and stores device data regarding the device state and device setting of each of a light source device and a control device forming an endoscope apparatus, an operation performed on the device, device error, and the like.

JP2001-327497A discloses that log data of a main body and measurement data of an acceleration sensor provided in an ultrasound probe are collected from an ultrasound diagnostic apparatus including the main body and the ultrasound probe connected to the main body and the log data and the measurement data are used for maintenance of the ultrasound diagnostic apparatus.

JP2006-331051A discloses that the operation amount such as the number of times of operation or the operation time of an endoscope is collected and the timing of replacing the endoscope with a new one or the like is determined based on the operation amount.
EP 2 698 096 A1 discloses an endoscope control device according to the preamble of claim 1. Identification information identifying an imaging apparatus is transmitted to a connector unit which transfers the identification data to a management server. Correction information, e.g. information for correcting pixel defects, is then transmitted from the management server to an imaging controller. Such procedure is set to facilitate the availability of correction information. This prior art document starts from a situation in which correction information is incorporate into the imaging apparatus prior to shipping the apparatus.

### SUMMARY OF THE INVENTION

The endoscope apparatus is used in a state in which a plurality of endoscopes are connected to the main body including a control device and a light source device. Since these endoscopes are repeatedly used, it is preferable that maintenance is appropriately performed. In the endoscope apparatus, there are a large number of combinations of the main body unit and the endoscopes. Therefore, in order to perform appropriate maintenance, it is important to be able to grasp the symptoms of troubles or failures that may be caused by the combinations.

JP2005-122707A and JP2006-043152A do not consider maintenance of endoscopes.

JP2001-327497A considers maintenance of an ultrasound probe that is not replaced for each examination, but does not consider maintenance of an endoscope used after being replaced for each examination.

In JP2006-331051A, the control device, the light source device, and the endoscope collect and manage the operation amount separately. However, even though the operation amount is collected in units of devices, it is not possible to know the symptoms of troubles or failures that may be caused by the combinations of the main body unit and the endoscopes.

The invention has been made in view of the above circumstances, and it is an object of the present invention to provide an endoscope control device, an endoscope maintenance support method, and a non-transitory computer readable recording medium storing an endoscope maintenance support program capable of appropriately performing maintenance of an endoscope.

An endoscope control device of the invention is defined in independent claim 1.

An endoscope maintenance support method of the invention is defined in independent claim 9.

A non-transitory computer readable recording medium storing an endoscope maintenance support program of the invention is defined in claim 16. Thus, it is possible to provide an endoscope control device, an endoscope maintenance support method, and a non-transitory computer readable recording medium storing an endoscope maintenance support program capable of appropriately performing maintenance of an endoscope.

### BRIEF DESCRIPTION OF THE DRAWINGS

Fig. 1 is a schematic diagram showing the schematic configuration of an endoscope apparatus maintenance management system including an endoscope apparatus that is an embodiment of the invention.
Fig. 2 is a schematic diagram showing the schematic configuration of the appearance of the endoscope apparatus in the maintenance management system shown in Fig. 1.
Fig. 3 is a schematic diagram showing the block configuration of the endoscope apparatus shown in Fig. 2.
Fig. 4 is a diagram showing the functional block of a system control unit shown in Fig. 3.

### DESCRIPTION OF THE PREFERRED EMBODIMENTS

Hereinafter, an embodiment of the invention will be described with reference to the diagrams.

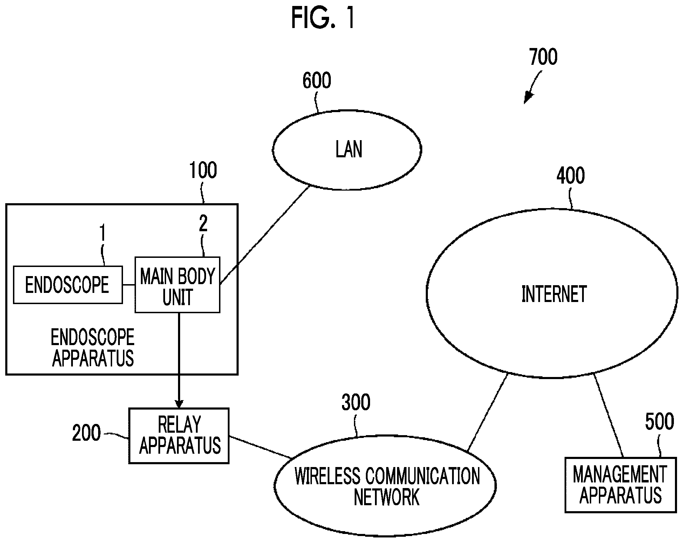

Fig. 1 is a schematic diagram showing the schematic configuration of an endoscope apparatus maintenance management system 700 including an endoscope apparatus 100 that is an embodiment of the invention.

The maintenance management system 700 includes the endoscope apparatus 100, a relay apparatus 200 forming an external apparatus, a wireless communication network 300, the Internet 400, a management apparatus 500 that is connected to the Internet 400 and is managed by an administrator who performs maintenance of the endoscope apparatus 100, and a local area network (LAN) 600 that forms a network provided in a medical facility where the endoscope apparatus 100 is installed.

Although the detailed configuration of the endoscope apparatus 100 will be described later, the endoscope apparatus 100 includes an endoscope 1 and a main body unit 2 that is connected to the endoscope 1 and controls the endoscope 1.

The main body unit 2 can be connected to the LAN 600 by wired communication or wireless communication. A medical management system for performing reservation management for endoscope examinations, management of examination images, cleaning management for the endoscope 1, and the like are connected to the LAN 600. The main body unit 2 can communicate with the relay apparatus 200 by wired communication or wireless communication.

The wireless communication network 300 is a communication network provided by a communication carrier, such as a mobile phone carrier. The wireless communication network 300 is connected to the Internet 400.

The relay apparatus 200 includes a communication interface for performing wired communication or wireless communication with the main body unit 2, a communication interface for connection to the wireless communication network 300, a processor for controlling communication using the two communication interfaces, a read only memory (ROM), and a random access memory (RAM).

The relay apparatus 200 stores transmission data (to be described later) received from the main body unit 2 of the endoscope apparatus 100 in the ROM, and transfers the transmission data to the management apparatus 500 through the wireless communication network 300 and the Internet 400.

As the relay apparatus 200, a personal computer, a smartphone, a tablet terminal, a mobile dedicated terminal, and the like are used. The relay apparatus 200 is communicably connected to the main body unit 2 of the endoscope apparatus 100 by a LAN cable, a universal serial bus (USB) cable, a USB connector, WiFi, or the like.

The management apparatus 500 includes a communication interface for connection to the Internet 400, a processor, a ROM, a RAM, and a database formed by a flash memory, a hard disk, or the like.

The processor of the management apparatus 500 stores and manages the transmission data transmitted from the relay apparatus 200 in the database. A worker who performs maintenance of the endoscope apparatus 100 obtains information for maintaining the endoscope apparatus 100 by analyzing the transmission data group stored in the database of the management apparatus 500.

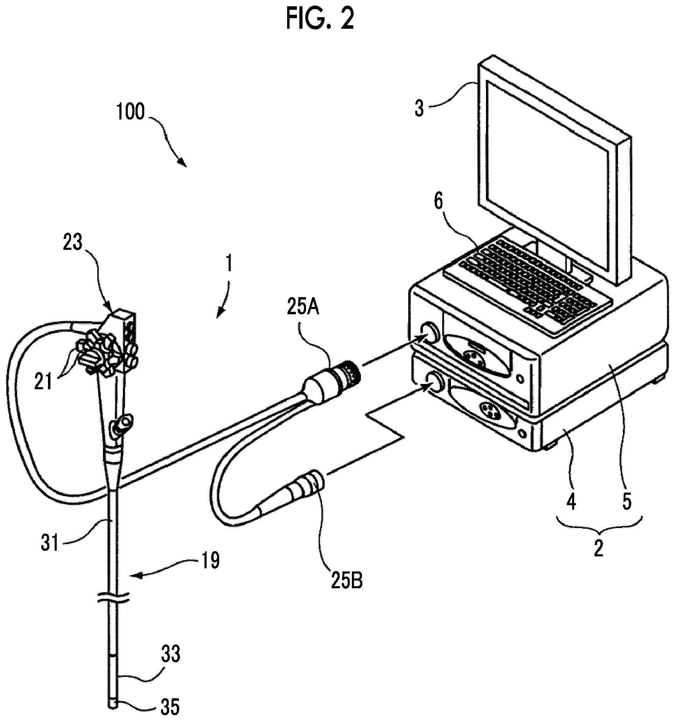

Fig. 2 is a schematic diagram showing the schematic configuration of the appearance of the endoscope apparatus 100 in the maintenance management system 700 shown in Fig. 1.

As shown in Fig. 2, the endoscope apparatus 100 includes the endoscope 1 and the main body unit 2, which is configured to include a control device 4 that is connected to the endoscope 1 and controls the endoscope 1 and a light source device 5 to which the endoscope 1 is connected.

A display unit 3 for displaying image information and the like and an input unit 6 for receiving an input operation are connected to the control device 4.

The endoscope 1 includes: an insertion part 19 that is inserted into a subject; an operation box 23 which is provided in a proximal end portion of the insertion part 19 and on which buttons for performing a mode switching operation, an imaging operation, an air and water supply operation, a suction operation, and the like are provided; an angle knob 21 that is provided adjacent to the operation box 23 in order to perform a bending operation on the distal end of the insertion part 19; a connector unit 25A for detachably connecting the endoscope 1 to the light source device 5; and a connector unit 25B for detachably connecting the endoscope 1 to the control device 4. An operation unit of the endoscope 1 is formed by the button group included in the operation box 23 and the angle knob 21.

Although not shown, various channels, such as a forceps channel into which a treatment instrument such as forceps is inserted, a channel for air supply and water supply, and a channel for suction, are provided inside the operation box 23 and the insertion part 19.

The insertion part 19 is configured to include a flexible portion 31 having flexibility, a bending portion 33 provided at the distal end of the flexible portion 31, and a distal end portion 35 provided at the distal end of the bending portion 33.

The bending portion 33 is formed so as to be bendable by the rotation operation of the angle knob 21. The bending portion 33 can be bent in any direction and at any angle according to a part of the subject or the like for which the endoscope 1 is used, so that the distal end portion 35 can be directed to a desired observation target part.

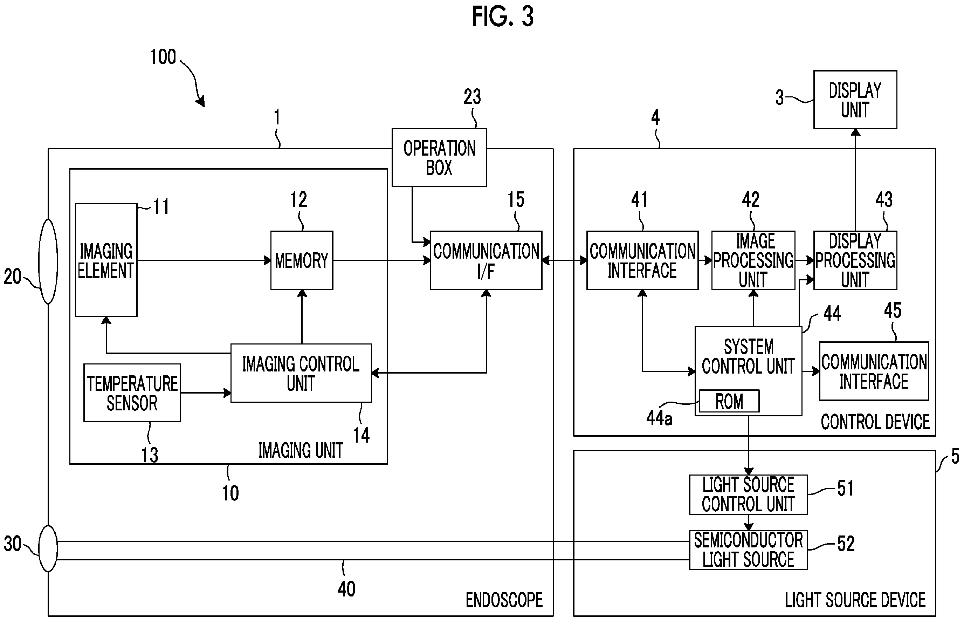

Fig. 3 is a schematic diagram showing the block configuration of the endoscope apparatus 100 shown in Fig. 2.

The endoscope 1 includes an imaging optical system 20 provided in the distal end portion 35, an imaging unit 10 for imaging an observation target part through the imaging optical system 20, an illumination window 30 provided in the distal end portion 35, a light guide 40 for guiding light to the illumination window 30, a communication interface (I/F) 15, and the above-described operation unit.

The light guide 40 extends from a position facing the illumination window 30 to the connector unit 25A, and guides light from the light source device 5 to the illumination window 30 in a state in which the connector unit 25A is attached to the light source device 5.

The communication interface 15 is a hardware interface for communicating with the control device 4. The communication interface 15 includes a power supply terminal for receiving power from the control device 4, a control signal terminal for receiving a control signal from the control device 4, a data signal terminal for transmitting various kinds of data to the control device 4, and the like.

The communication interface 15 transmits an operation signal, which is input according to the operation of the various buttons included on the operation box 23, to the control device 4. The communication interface 15 transmits a captured image signal stored in a memory 12 to the control device 4 under the control of an imaging control unit 14. In addition, the control signal received by the communication interface 15 is input to the imaging control unit 14.

The imaging unit 10 includes an imaging element 11, the memory 12 such as a RAM, a temperature sensor 13, and the imaging control unit 14.

The temperature sensor 13 is a temperature detection unit that detects the temperature of the imaging element 11. For example, a thermal diode can be used.

The imaging control unit 14 controls the imaging element 11 according to the control signal input from the communication interface 15 to image the observation target part. The captured image signal obtained by the imaging of the imaging element 11 is stored in the memory 12.

The imaging control unit 14 performs control to transmit the captured image signal stored in the memory 12 to the control device 4 through the communication interface 15.

The imaging control unit 14 performs control to transmit the temperature information detected by the temperature sensor 13 to the control device 4 through the communication interface 15.

The light source device 5 includes a light source control unit 51 and a semiconductor light source 52.

The semiconductor light source 52 is a light source for generating light to be emitted to the observation target part. The light emitted from the semiconductor light source 52 is introduced into the light guide 40 in the connector unit 25A, and is emitted to the observation target part through the illumination window 30.

The light source control unit 51 controls the light emission timing and the like of the semiconductor light source 52.

The control device 4 includes a communication interface 41, an image processing unit 42, a display processing unit 43, a system control unit 44, and a communication interface 45.

The communication interface 41 is a hardware interface that is connected to the communication interface 15 of the endoscope 1 to communicate with the endoscope 1.

The communication interface 41 includes a power supply terminal for supplying power to the endoscope 1, a control signal terminal for transmitting a control signal for controlling the imaging control unit 14 of the endoscope 1, a data signal terminal for receiving the captured image signal from the endoscope 1, and the like.

The image processing unit 42 includes a memory for temporarily storing the captured image signal received by the communication interface 41 and a processor. The processor generates captured image data by performing image processing on the captured image signal of one frame of the motion picture stored in the memory.

The display processing unit 43 generates display data from the captured image data generated by the image processing unit 42, and displays an image based on the display data on the display unit 3. The image processing unit 42 and the display processing unit 43 are controlled by the system control unit 44.

The communication interface 45 is a hardware interface for communicating with the relay apparatus 200 by wireless communication or wired communication.

As the communication interface 45, for example, a wired LAN module conforming to the IEEE 802.3 standard, a wireless LAN module conforming to the IEEE 802.11 standard, and a communication module conforming to the USB standard are used.

The system control unit 44 includes various processors (not shown), a ROM 44a forming a storage medium, and a RAM (not shown). The processor of the system control unit 44 performs overall control of the entire endoscope apparatus 100 by executing the program stored in the ROM 44a. The program includes a maintenance support program.

The various processors include: a central processing unit (CPU) that is a general-purpose processor for performing various kinds of processing by executing a program; a programmable logic device (PLD) that is a processor whose circuit configuration can be changed after manufacture, such as field programmable gate array (FPGA), a dedicated electrical circuit that is a processor having a dedicated circuit configuration for executing specific processing, such as application specific integrated circuit (ASIC); and the like.

More specifically, the structure of each of the various processors is an electrical circuit in which circuit elements, such as semiconductor elements, are combined.

The processor of the system control unit 44 may be configured to include one of various processors, or may be a combination of two or more processors of the same type or different types (for example, a combination of a plurality of FPGAs or a combination of a CPU and an FPGA).

Based on the operation signal of the button of the operation box 23 received from the endoscope 1, the processor of the system control unit 44 performs processing corresponding to the operation signal (imaging control, light source control, electrical control of various channels included in the endoscope 1, and the like).

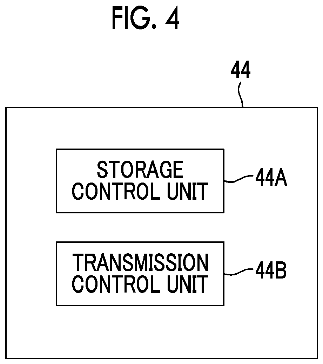

Fig. 4 is a diagram showing the functional block of the system control unit 44 shown in Fig. 3. The processor of the system control unit 44 functions as a storage control unit 44A and a transmission control unit 44B by executing the program stored in the ROM 44a.

The storage control unit 44A stores, in the ROM 44a, transmission data including identification information of an examination performed using the endoscope 1 connected to the control device 4 and state information indicating the state of hardware (specifically, the imaging element 11, the operation unit (each button of the operation box 23 and the angle knob 21), and the communication interface 15) included in the endoscope 1 under the examination.

The identification information of the examination is information that is a combination of the serial number of the endoscope 1 input to the control device 4 at the start of examination and the start date of examination, information that is a combination of symbols and numbers issued from a medical management system cooperating with the endoscope apparatus 100, and the like.

The state information indicating the state of the communication interface 15 under examination includes information regarding the history of communication with the communication interface 15 under examination, information regarding the state of power supply to the communication interface 15 under examination, and the like.

The state information indicating the state of the imaging element 11 under examination includes the temperature information of the imaging element 11 under examination detected by the temperature sensor 13, information indicating the number and positions of defective pixels included in a number of imaging pixels forming the imaging surface of the imaging element 11, and the like.

In order to obtain the information of defective pixels, for example, a medical worker performs an operation of imaging a test pattern, such as a white image or a black image, using the imaging element 11 before starting the examination, and the image processing unit 42 performs processing for detecting the number and positions of defective pixels based on the captured image signal obtained by the imaging.

The state information indicating the state of the operation unit under examination includes information regarding the operation history of each button included on the operation box 23 (which button has been pressed how many times), the operation history of the angle knob 21 (how many times the angle knob 21 has rotated in which direction), and the like.

For example, by arranging a sensor that detects the rotation position of the angle knob 21 in the vicinity of the angle knob 21, the operation history of the angle knob 21 can be acquired based on information from the sensor.

The storage control unit 44A may cooperate with a cleaning management system that performs cleaning management of the endoscope 1 and include information of the cumulative number of cleaning at the start of examination of the endoscope 1 in the transmission data described above. Information of the cumulative value of the number of times of power supply through the communication interface 15 of the endoscope 1 may be included in the transmission data.

The transmission control unit 44B transmits the transmission data stored in the ROM 44a by the storage control unit 44A to the relay apparatus 200 through the communication interface 45 after the end of the examination.

Upon receiving the transmission data transmitted from the control device 4, the relay apparatus 200 stores the transmission data in the ROM 44a. Then, the relay apparatus 200 transmits the transmission data to the management apparatus 500 at a certain timing, such as a timing at which the traffic of the wireless communication network 300 is small.

With the above system configuration, every time an examination is performed using the endoscope apparatus 100, transmission data including the state information indicating the state of the endoscope 1 under examination and the identification information of the examination is accumulated in the database of the management apparatus 500.

In this manner, the state information of the hardware of the endoscope 1 is stored in the database of the management apparatus 500 in units of examinations. Therefore, for each examination, it is possible for the worker to grasp how the endoscope 1 has operated.

Since it is possible to analyze the operation in units of examinations as described above, even in a case where a plurality of endoscopes 1 are connected to the main body unit 2 to perform an examination in the same manner as in the endoscope apparatus 100, it is possible to grasp the operation state of the apparatus for each combination of the main body unit 2 and the endoscope 1.

Therefore, for example, it is also possible to know the symptoms of troubles or failures that may be caused by the combination of the main body unit and the endoscope. As a result, appropriate maintenance can be performed.

In addition, by counting transmission data having the same serial number included in the identification information among the pieces of transmission data stored in the database of the management apparatus 500, it is also possible to obtain information of the cumulative number of times the button has been pressed until then, the number of times the scope has been used, and the like for the endoscope 1 having a certain serial number. Therefore, it is possible to perform appropriately maintenance by further using the information.

In the maintenance management system 700, transmission data is transmitted from the endoscope apparatus 100 to the relay apparatus 200, and is transmitted from the relay apparatus 200 to the management apparatus 500. Since the relay apparatus 200 can be operated without being connected to the LAN 600 satisfying the high security required for the medical facility, it is possible to reduce the cost required for constructing the system.

It is preferable that the storage control unit 44A stores not only the identification information and the state information but also the operation history information of the control device 4 under examination, as the transmission data, in the ROM 44a.

The operation history information includes information regarding the control history of the light source device 5 by the processor of the system control unit 44, the control history of the endoscope 1 by the processor of the system control unit 44, the signal processing history by the image processing unit 42, the notification history of error made through the display unit 3.

As described above, since the operation history information of the control device 4 under examination is included in the transmission data, the operation analysis of the endoscope apparatus 100 can be performed in more detail. As a result, appropriate maintenance can be performed.

In order to secure the capacity of the ROM 44a, it is preferable that, after completing the transmission of the transmission data stored in the ROM 44a to the relay apparatus 200, the transmission control unit 44B of the control device 4 deletes the transmission data from the ROM 44a or releases a region where the transmission data is stored as a storage region for storing new transmission data.

However, the transmission control unit 44B of the control device 4 may leave some information included in the transmission data without deleting the information from the ROM 44a.

For example, information regarding the history of communication with the communication interface 15 included in the transmission data may be left in the ROM 44a. In a case where the worker performs maintenance work in a place where the endoscope apparatus 100 is installed, some of the information remains on the apparatus side as described above, so that the information can be used for the maintenance work.

After transmitting the transmission data to the relay apparatus 200, the transmission control unit 44B of the control device 4 may store examination identification information included in the transmission data and transmission completion information, which indicates that the transmission of the transmission data has been completed, in the ROM 44a so as to be associated with each other.

As a result, the worker who performs the maintenance work in the place where the endoscope apparatus 100 is installed can check whether or not the transmission data has been correctly transmitted. In a case where trouble occurs in a transmission data transmission function, repair or the like relevant to the transmission function can be performed.

The transmission data generated by the storage control unit 44A may include state information indicating the state of at least one of the imaging element 11, each button of the operation box 23, the angle knob 21, or the communication interface 15. Among the pieces of state information, the state information of the communication interface 15 includes information of error and the like that may occur in the combination of the main body unit 2 and the endoscope 1. This is effective for appropriate maintenance.

(14) The endoscope maintenance support method described in (13), in which the state information of the imaging element includes at least one of temperature information of the imaging element or defective pixel information of the imaging element.

(15) The endoscope maintenance support method described in any one of (10) to (14), in which, in the storage control step, not only the identification information and the state information but also operation history information of the control device under the examination

### Explanation of References

100: endoscope apparatus
200: relay apparatus
300: wireless communication network
400: Internet
500: management apparatus
600: LAN
700: maintenance management system
1: endoscope
10: imaging unit
11: imaging element
12: memory
13: temperature sensor
14: imaging control unit
15: communication interface
2: main body unit
3: display unit
4: control device
41: communication interface
42: image processing unit
43: display processing unit
44: system control unit
44a: ROM
45: communication interface
5: light source device
51: light source control unit
52: semiconductor light source
6: input unit
19: insertion part
20: imaging optical system
21: angle knob
23: operation box
25A, 25B: connector unit
30: illumination window
31: flexible portion
33: bending portion
35: distal end portion
40: light guide

## Claims

1. An endoscope control device (4), comprising:
a storage control unit (44A) that stores transmission data, in a storage medium;
a communication interface (41); and
a transmission control unit (44B) that transmits the transmission data to an external apparatus through the communication interface, **characterized in that** the storage control unit (44A) is adapted to store transmission data including identification information of an examination performed using an endoscope and state information, which indicates a state of hardware included in the endoscope (1) under the examination, wherein the hardware includes an operation unit included in the endoscope (1),
and wherein the state information is information regarding a operation history of each button included on an operation box of the endoscope or a operation history of an angle knob of the endoscope.

2. The endoscope control device (4) according to claim 1,
wherein the hardware includes a communication interface of the endoscope (1) for communicating with the control device (4).

3. The endoscope control device (4) according to claim 1 or 2,
wherein the hardware includes an imaging element (11).

4. The endoscope control device (4) according to claim 3,
wherein the state information of the imaging element (11) includes at least one of temperature information of the imaging element or defective pixel information of the imaging element.

5. The endoscope control device (4) according to any one of claims 1 to 4,
wherein the storage control unit (44A) stores not only the identification information and the state information but also operation history information of the control device under the examination, as the transmission data, in the storage medium.

6. The endoscope control device (4) according to any one of claims 1 to 5,
wherein the transmission data is transmitted from the external apparatus to a management apparatus through a wireless network different from a network connected to the control device (4).

7. The endoscope control device (4) according to claim 1,
wherein the storage control unit (44A) stores the transmission data including state information which indicates a state of an operation unit included in the endoscope under the examination, as the state information, in a storage medium.

8. The endoscope control device (4) according to claim 1,
wherein the storage control unit (44A) stores the transmission data including state information which indicates a state of an imaging element included in the endoscope under the examination, as the state information, in a storage medium.

9. An endoscope maintenance support method, comprising:
a storage control step of storing transmission data including identification information of an examination performed using an endoscope and state information, which indicates a state of hardware included in the endoscope (1) under the examination, in a storage medium of an endoscope control device (4) to which the endoscope is connected; and
a transmission control step of transmitting the transmission data to an external apparatus through a communication interface (41) included in the control device (4),
wherein the hardware includes an operation unit included in the endoscope (1),
and wherein the state information is information regarding a operation history of each button included on an operation box of the endoscope or a operation history of an angle knob of the endoscope.

10. The endoscope maintenance support method according to claim 9,
wherein the hardware includes an operation unit included in the endoscope (1).

11. The endoscope maintenance support method according to claim 9 or 10,
wherein the hardware includes a communication interface (15) of the endoscope (1) for communicating with the control device (4).

12. The endoscope maintenance support method according to any one of claims 9 to 11,
wherein the hardware includes an imaging element (11).

13. The endoscope maintenance support method according to claim 12,
wherein the state information of the imaging element (11) includes at least one of temperature information of the imaging element or defective pixel information of the imaging element (11).

14. The endoscope maintenance support method according to any one of claims 9 to 13,
wherein, in the storage control step, not only the identification information and the state information but also operation history information of the control device (4) under the examination is stored in the storage medium as the transmission data.

15. The endoscope maintenance support method according to any one of claims 9 to 14,
wherein the transmission data is transmitted from the external apparatus to a management apparatus through a wireless network different from a network connected to the control device (4).

16. A non-transitory computer readable recording medium storing an endoscope maintenance support program causing a computer to execute:
a storage control step of storing transmission data including identification information of an examination performed using an endoscope and state information, which indicates a state of hardware included in the endoscope under the examination, in a storage medium of an endoscope control device (4) to which the endoscope is connected; and
a transmission control step of transmitting the transmission data to an external apparatus through a communication interface included in the control device (4),
wherein the hardware includes an operation unit included in the endoscope (1),
and wherein the state information is information regarding a operation history of each button included on an operation box of the endoscope or a operation history of an angle knob of the endoscope

17. The endoscope control device according to any one of claims 1 to 9,
wherein the identification information of the examination is information that is a combination of a serial number of the endoscope and a start date of the examination or information that is a combination of symbols and numbers issued from a medical management system cooperating with the endoscope.

18. The endoscope control device according to any one of claims 1 to 9,
wherein the state information is information regarding a history of communication with a communication interface of the endoscope under examination or information regarding a state of power supply to the communication interface under examination.

19. The endoscope control device according to claim 5,
wherein the state information of the imaging element is the temperature information of the imaging element.

## Patentansprüche

1. Endoskopsteuervorrichtung (4), umfassend:
eine Speichersteuereinheit (44A), die in einem Speichermedium Senddaten speichert;
eine Kommunikationsschnittstelle (41); und
eine Sendesteuereinheit (44B), die Sendedaten über die Kommunikationsschnittstelle an eine externe Vorrichtung sendet, **dadurch gekennzeichnet, dass** die Speichersteuereinheit (44A) ausgebildet ist zum Speichern von Sendedaten, die Kennungsinformation einer Untersuchung enthalten, die mittels eines Endoskops ausgeführt wurde, und Statusinformation enthalten, wobei letztere einen Zustand von Hardware in dem in der Untersuchung befindlichen Endoskop (1) angibt, wobei die Hardware eine Bedieneinheit in dem Endoskop (1) enthält,
und wobei die Statusinformation Information bezüglich einer Bedienungsgeschichte jeder Taste ist, die in einer Bedienbox des Endoskops enthalten ist, oder eine Bedienungsgeschichte eines Winkelknopfs des Endoskops.

2. Endoskopsteuervorrichtung (4) nach Anspruch 1,
bei der die Hardware eine Kommunikationsschnittstelle des Endoskops (1) zur Kommunikation mit der Steuervorrichtung (4) enthält.

3. Endoskopsteuervorrichtung (4) nach Anspruch 1 oder 2,
bei der die Hardware ein Bildgebungselement (11) enthält.

4. Endoskopsteuervorrichtung (4) nach Anspruch 3,
bei der die Statusinformation des Bildelements (11) mindestens Temperaturinformation des Bildelements und/oder Information über defekte Pixel des Bildelements enthält.

5. Endoskopsteuervorrichtung (4) nach einem der Ansprüche 1 bis 4,
bei der die Speichersteuereinheit (44A) nicht nur die Kennungsinformation und die Statusinformation speichert, sondern außerdem Bedienungsgeschichte-Information der in der Untersuchung befindlichen Steuervorrichtung als Sendedaten in dem Speichermedium speichert.

6. Endoskopsteuervorrichtung (4) nach einem der Ansprüche 1 bis 5,
bei der die Sendedaten von der externen Vorrichtung zu einer Verwaltungsvorrichtung über ein Drahtlosnetzwerk gesendet werden, welches verschieden ist von einem an die Steuervorrichtung (4) angeschlossenem Netzwerk.

7. Endoskopsteuervorrichtung (4) nach Anspruch 1,
bei der die Speichersteuereinheit (44A) in einem Speichermedium die Sendedaten einschließlich Statusinformation speichert, welche einen Zustand einer in dem in der Untersuchung befindlichen Endoskop enthaltenen Bedieneinheit als die Statusinformation angibt.

8. Endoskopsteuervorrichtung (4) nach Anspruch 1,
bei der die Speichersteuereinheit (44A) die Sendedaten einschließlich Statusinformation in einem Speichermedium speichert, wobei die Statusinformation einen Zustand eines in dem in der Untersuchung befindlichen Endoskop enthaltenen Bildgebungselement als die Statusinformation angibt.

9. Endoskop-Wartungsunterstützungsverfahren, umfassend:
einen Speichersteuerschritt des Speicherns von Sendedaten einschließlich Kennungsinformation einer mittels eines Endoskops ausgeführten Untersuchung und Statusinformation, die einen Zustand einer in dem in der Untersuchung begriffenen Endoskop (1) enthaltenen Hardware angibt, in einem Speichermedium einer Endoskopsteuervorrichtung (4), an die das Endoskop angeschlossen ist; und
einen Sendesteuerschritt des Sendens der Sendedaten zu einer externen Vorrichtung über eine in der Steuereinheit (4) enthaltene Kommunikationsschnittstelle (41),
wobei die Hardware eine in dem Endoskop (1) enthaltene Bedieneinheit beinhaltet,
und wobei die Statusinformation Information bezüglich einer Bediengeschichte jeder Taste ist, die in einer Bedienbox des Endoskops enthalten ist, oder einer Bediengeschichte eines Winkelknopfs des Endoskops.

10. Verfahren nach Anspruch 9,
bei dem die Hardware eine Bedieneinheit in dem Endoskop (1) enthält.

11. Verfahren nach Anspruch 9 oder 10,
bei dem die Hardware eine Kommunikationsschnittstelle (15) des Endoskops (1) zum Kommunizieren mit der Steuervorrichtung (4) enthält.

12. Verfahren nach einem der Ansprüche 9 bis 11,
bei dem die Hardware ein Bildgebungselement (11) enthält.

13. Verfahren nach Anspruch 12,
bei dem die Statusinformation des Bildelements (11) mindestens Temperaturinformation des Bildelements und/oder Information über defekte Pixel des Bildelements enthält.

14. Verfahren nach einem der Ansprüche 9 bis 13,
bei dem in dem Speichersteuerschritt nicht nur die Kennungsinformation und die Statusinformation als Sendedaten in dem Speichermedium gespeichert werden, sondern auch Bediengeschichte-Information der in der Untersuchung befindlichen Steuervorrichtung (4).

15. Verfahren nach einem der Ansprüche 9 bis 14,
bei dem die Sendedaten von der externen Vorrichtung über ein Drahtlosnetzwerk zu einer Verwaltungsvorrichtung über ein Drahtlosnetzwerk gesendet werden, das verschieden ist von einem mit der Steuervorrichtung (4) verbundenen Netzwerk.

16. Nicht-flüchtiges, computer-lesbares Aufzeichnungsmedium, das ein Endoskop-Wartungsunterstützungsprogramm speichert, das einen Computer veranlasst, Folgendes auszuführen:
einen Speichersteuerschritt des Speicherns von Sendedaten einschließlich Kennungsinformation einer mittels eines Endoskops ausgeführten Untersuchung und Statusinformation, die einen Zustand einer indem Endoskop einer in der Untersuchung begriffenen Endoskop (1) enthaltenen Hardware angibt, in einem Speichermedium einer Endoskopsteuervorrichtung (4) an die das Endoskop angeschlossen ist; und
einen Sendesteuerschritt des Sendens der Sendedaten zu einer externen Vorrichtung über eine in der Steuereinheit (4) enthaltene Kommunikationsschnittstelle (41),
wobei die Hardware eine in dem Endoskop (1) enthaltene Bedieneinheit beinhaltet,
und wobei die Statusinformation Information bezüglich einer Bediengeschichte jeder Taste ist, die in einer Bedienbox des Endoskops enthalten ist, oder Bediengeschichte eines Winkelknopfs des Endoskops.

17. Endoskopsteuervorrichtung nach einem der Ansprüche 1 bis 9,
bei dem die Kennungsinformation der Untersuchung Information ist, bei der es sich um eine Kombination aus einer Seriennummer des Endoskops und eines Startdatums der Untersuchung ist, oder Information, bei der es sich um eine Kombination von Symbolen und Zahlen handelt, die von einem medizinischen Verwaltungssystem ausgegeben werden, das mit dem Endoskop zusammenarbeitet.

18. Endoskopsteuervorrichtung nach einem der Ansprüche 1 bis 9,
bei der die Statusinformation Information bezüglich einer Geschichte der Kommunikation mit einer Kommunikationsschnittstelle des in der Untersuchung befindlichen Endoskops ist, oder Information bezüglich eines Zustands der Stromversorgung für die unter einer Untersuchung befindlichen Kommunikations-Schnittstelle.

19. Endoskopsteuervorrichtung nach Anspruch 5,
bei der die Statusinformation des Bildgebungselements die Temperaturinformation des Bildelements ist.

## Revendications

1. Dispositif de commande d'endoscope (4), comprenant :
une unité de commande de stockage (44A), laquelle stocke des données de transmission, sur un support de stockage ;
une interface de communication (41), et
une unité de commande de transmission (44B), laquelle transmet des données de transmission à un appareil externe par le biais de l'interface de communication, **caractérisé en ce que** l'unité de commande de stockage (44A) est apte à stocker des données de transmission incluant des informations d'identification d'un examen réalisé à l'aide d'un endoscope et des informations d'état, lesquelles indiquent un état de matériel inclus dans l'endoscope (1) examiné, dans lequel le matériel inclut une unité de fonctionnement incluse dans l'endoscope (1), et
dans lequel les informations d'état sont des informations concernant un historique de fonctionnement de chaque bouton inclus dans un boîtier de fonctionnement de l'endoscope ou un historique de fonctionnement d'un bouton d'angle de l'endoscope.

2. Dispositif de commande d'endoscope (4) selon la revendication 1,
dans lequel le matériel inclut une interface de communication de l'endoscope (1) pour communiquer avec le dispositif de commande (4).

3. Dispositif de commande d'endoscope (4) selon la revendication 1 ou 2,
dans lequel le matériel inclut un élément d'imagerie (11).

4. Dispositif de commande d'endoscope (4) selon la revendication 3,
dans lequel les informations d'état de l'élément d'imagerie (11) incluent au moins un élément parmi des informations de température de l'élément d'imagerie ou des informations de pixel défectueux de l'élément d'imagerie.

5. Dispositif de commande d'endoscope (4) selon l'une quelconque des revendications 1 à 4,
dans lequel l'unité de commande de stockage (44A) stocke non seulement les informations d'identification et les informations d'état, mais également des informations d'historique de fonctionnement du dispositif de commande examiné, comme données de transmission, dans le support de stockage.

6. Dispositif de commande d'endoscope (4) selon l'une quelconque des revendications 1 à 5,
dans lequel les données de transmission sont transmises de l'appareil externe vers un appareil de gestion par le biais d'un réseau sans fil différent d'un réseau connecté au dispositif de commande (4).

7. Dispositif de commande d'endoscope (4) selon la revendication 1,
dans lequel l'unité de commande de stockage (44A) stocke les données de transmission incluant des informations d'état, lesquelles indiquent un état d'une unité de fonctionnement incluse dans l'endoscope examiné, comme informations d'état, dans un support de stockage.

8. Dispositif de commande d'endoscope (4) selon la revendication 1,
dans lequel l'unité de commande de stockage (44A) stocke les données de transmission incluant des informations d'état, lesquelles indiquent un état d'un élément d'imagerie inclus dans l'endoscope examiné, comme informations d'état, dans un support de stockage.

9. Procédé de prise en charge de maintenance d'endoscope, comprenant les étapes suivantes :
une étape de commande de stockage pour stocker des données de transmission incluant des informations d'identification d'un examen réalisé à l'aide d'un endoscope et des informations d'état, lesquelles indiquent un état de matériel inclus dans l'endoscope (1) examiné, dans un support de stockage d'un dispositif de commande d'endoscope (4) auquel est connecté l'endoscope ;
une étape de commande de transmission pour transmettre les données de transmission vers un appareil externe par le biais d'une interface de communication (41) incluse dans le dispositif de commande (4), et
dans lequel le matériel inclut une unité de fonctionnement incluse dans l'endoscope (1), et
dans lequel les informations d'état sont des informations concernant un historique de fonctionnement de chaque bouton inclus dans un boîtier de fonctionnement de l'endoscope ou un historique de fonctionnement d'un bouton d'angle de l'endoscope.

10. Procédé de prise en charge de maintenance d'endoscope selon la revendication 9,
dans lequel le matériel inclut une unité de fonctionnement incluse dans l'endoscope (1).

11. Procédé de prise en charge de maintenance d'endoscope selon la revendication 9 ou 10,
dans lequel le matériel inclut une interface de communication (15) de l'endoscope (1) pour communiquer avec le dispositif de commande (4).

12. Procédé de prise en charge de maintenance d'endoscope selon l'une quelconque des revendications 9 à 11,
dans lequel le matériel inclut un élément d'imagerie (11).

13. Procédé de prise en charge de maintenance d'endoscope selon la revendication 12,
dans lequel les informations d'état de l'élément d'imagerie (11) incluent au moins un élément parmi des informations de température de l'élément d'imagerie ou des informations de pixel défectueux de l'élément d'imagerie (11).

14. Procédé de prise en charge de maintenance d'endoscope selon l'une quelconque des revendications 9 à 13,
dans lequel lors de l'étape de commande de stockage, non seulement les informations d'identification et les informations d'état, mais également des informations d'historique de fonctionnement du dispositif de commande (4) examiné, sont stockées dans le support de stockage comme données de transmission.

15. Procédé de prise en charge de maintenance d'endoscope selon l'une quelconque des revendications 9 à 14,
dans lequel les données de transmission sont transmises de l'appareil externe vers un appareil de gestion par le biais d'un réseau sans fil différent d'un réseau connecté au dispositif de commande (4).

16. Support d'enregistrement lisible par ordinateur non transitoire stockant un programme de prise en charge de maintenance d'endoscope faisant en sorte qu'un ordinateur exécute les étapes suivantes :
une étape de commande de stockage pour stocker des données de transmission incluant des informations d'identification d'un examen réalisé à l'aide d'un endoscope et des informations d'état, lesquelles indiquent un état de matériel inclus dans l'endoscope examiné, dans un support de stockage d'un dispositif de commande d'endoscope (4) auquel est connecté l'endoscope, et
une étape de commande de transmission pour transmettre les données de transmission vers un appareil externe par le biais d'une interface de communication incluse dans le dispositif de commande (4) ;
dans lequel le matériel inclut une unité de fonctionnement incluse dans l'endoscope (1), et
dans lequel les informations d'état sont des informations concernant un historique de fonctionnement de chaque bouton inclus dans un boîtier de fonctionnement de l'endoscope ou un historique de fonctionnement d'un bouton d'angle de l'endoscope.

17. Dispositif de commande d'endoscope selon l'une quelconque des revendications 1 à 9,
dans lequel les informations d'identification de l'examen sont des informations qui combinent un numéro de série de l'endoscope et une date de début de l'examen ou des informations qui combinent des symboles et chiffres émis à partir d'un système de gestion médical coopérant avec l'endoscope.

18. Dispositif de commande d'endoscope selon l'une quelconque des revendications 1 à 9,
dans lequel les informations d'état sont des informations concernant un historique de communication avec une interface de communication de l'endoscope examiné ou des informations concernant un état d'alimentation électrique vers l'interface de communication examinée.

19. Dispositif de commande d'endoscope selon la revendication 5,
dans lequel les informations d'état de l'élément d'imagerie sont les informations de température de l'élément d'imagerie.
